(19) Europäisches Patentamt
European Patent Office
Office européen des brevets

(11) Numéro de publication: **0 068 564**
**B1**

(12) **FASCICULE DE BREVET EUROPEEN**

(45) Date de publication du fascicule du brevet: **09.04.86**

(51) Int. Cl.⁴: **C 07 D 301/12** // C07D303/04, C07D493/04

(21) Numéro de dépôt: **82200745.6**

(22) Date de dépôt: **16.06.82**

(54) **Procédé pour la fabrication d'époxydes.**

(30) Priorité: **26.06.81 FR 8112797**

(43) Date de publication de la demande:
**05.01.83 Bulletin 83/1**

(45) Mention de la délivrance du brevet:
**09.04.86 Bulletin 86/15**

(84) Etats contractants désignés:
**AT BE CH DE FR GB IT LI NL SE**

(56) Documents cités:
**FR - A - 1 201 458**
**FR - A - 1 506 459**
**FR - A - 2 302 302**
**FR - A - 2 378 773**
**US - A - 4 242 285**

**Le dossier contient des informations techniques présentées postérieurement au dépôt de la demande et ne figurant pas dans le présent fascicule.**

(73) Titulaire: **INTEROX Société anonyme dite:, Rue du Prince Albert, 33, B-1050 Bruxelles (BE)**

(72) Inventeur: **Lecloux, André, Van Immersseellaan 15, B-1860 Meise (BE)**
Inventeur: **Legrand, Franz, Rue des Vaches, 71, B-7300 Quaregnon (BE)**
Inventeur: **Declerck, Claude, Avenue des Croix de Guerre, 128, B-1120 Bruxelles (BE)**

(74) Mandataire: **Lederer, Franz, Dr. et al, Patentanwälte Dr. Lederer Franz Meyer-Roxlau Reiner F. Lucile-Grahn-Strasse 22, D-8000 München 80 (DE)**

**EP 0 068 564 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

La présente invention concerne un procédé pour la fabrication d'époxydes en faisant réagir du peroxyde d'hydrogène avec un composé contenant une double liaison éthylénique a l'intervention d'un catalyseur.

Les époxydes sont des produits présentant de nombreuses applications industrielles, par exemple dans la fabrication de polymères thermodurcissables utilisables comme adhésifs ou revêtements de surface notamment. Industriellement, les époxydes sont fabriqués en général selon des procédés complexes tels que la déshydrochloration des chlorhydrines correspondantes ou l'oxydation catalytique des oléfines par des hydroperoxydes ou directement par l'oxygène moléculaire. Pour réduire les coûts de fabrication et la formation de sous-produits et simplifier les procédés, on a proposé de fabriquer les époxydes en faisant réagir directement du peroxyde d'hydrogène avec l'oléfine correspondante en phase liquide grâce à la mise en oeuvre de certains catalyseurs spécifiques. Les procédés connus à ce jour ont cependant tous des inconvénients divers. La productivité des réacteurs est faible car la réaction est trop lente ou les solutions à mettre en oeuvre sont trop diluées, les catalyseurs ont une durée de vie insuffisante et sont rapidement désactivés, ou la sélectivité est mauvaise et on obtient beaucoup de sous-produits indésirables tels que des diols ou des alkoxyalcools ou encore la séparation des produits de la réaction est malaisée. De plus, le peroxyde d'hydrogène doit souvent être mis en oeuvre sous forme anhydre et le mélange réactionnel doit être maintenu anhydre. Certains catalyseurs ont en outre une activité qui dépend fortement de l'oléfine à époxyder. Ainsi, lorsqu'on utilise l'oxyde de sélénium comme catalyseur on obtient des diols avec des oléfines telles que le cyclohexène et des époxydes uniquement avec des oléfines très complexes de type macrocyclique (J. Itakura et coll., Bull. of the Chem. Soc. of Japan, 1969, 42, p. 1604 à 1608).

Le brevet FR—A—1 506 459 (Naphta Chimie) concerne un procédé pour l'époxydation des oléfines par le peroxyde d'hydrogène selon lequel on opère en présence d'une base organique azotée et d'un composé de certains métaux des Groupes 6b (molybdène, tungstène, uranium), 5b (vanadium, niobium, tantale) ou 7b (rhénium) du Tableau Périodique des Elements (Classification internationale).

Le brevet FR—A—1 201 458 (Columbia-Southern Chem. Corp.) concerne un procéde pour l'époxydation des oléfines par le peroxyde d'hydrogène selon lequel on emploie, comme catalyseurs, des peracides d'oxydes métalliques, on opère en milieu aqueux et on maintient le pH à une valeur inférieure à 7 et au moins égale à 3.

La demande de brevet FR—A—2 302 302 (Union Carbide) concerne un procédé pour l'époxydation des oléfines par le peroxyde d'hydrogène selon lequel on met en oeuvre, comme catalyseur, de l'arsenic élémentaire ou un composé de l'arsenic et on opère dans un solvant organique inerte.

Dans le brevet US—A—4 242 285 (J. M. Renga), on divulgue l'époxydation des oléfines par le peroxyde d'hydrogène en présence de dérivés particuliers du sélénium dans un mélange réactionnel contenant une phase aqueuse et une phase organique. Le solvant organique mis en oeuvre peut être choisi parmi les alkanes chlorés, les composés aromatiques, les composés hétérocycliques dont la pyridine et les oléfines: seuls le chlorure de méthylène et le 1,2-dichloréthane sont décrits dans les exemples.

La demande de brevet FR—A—2 378 773 (PUK) concerne également l'emploi de catalyseurs à base d'arsenic pour époxyder les oléfines. On y renseigne que la réaction peut être effectuée en présence de tampons tels que la pyridine, les phosphates alcalins, une amine tertiaire ou les acétates alcalins.

La présente invention a pour but de pallier les inconvénients des procédés connus et de fournir un procédé qui donne une sélectivité élevée en époxyde avec une bonne productivité.

L'invention concerne à cet effet un procédé pour la fabrication d'époxydes selon lequel on fait réagir du peroxyde d'hydrogène avec un composé contenant une double liaison éthylénique dans un mélange réactionnel homogène liquide contenant un catalyseur et une base et selon lequel on maintient la concentration en eau du mélange réactionnel à moins de 10% en poids, on utilise une base choisie parmi les bases organiques azotées ayant un pKa ne dépassant pas 8 et on utilise un catalyseur choisi parmi les composés du sélénium.

Dans le procédé selon l'invention, le degré d'oxydation du sélénium n'est pas critique. De préférence, on met en oeuvre des composés du sélénium dans lesquels le sélénium se trouve à un degré d'oxydation inférieur au degré d'oxydation maximum.

Les composés du sélénium utilisés comme catalyseurs sont choisis en général parmi les composés solubles dans le mélange réactionnel aux concentrations utilisées. Ils peuvent être de nature organique ou inorganique. Comme composés inorganiques, on peut ainsi utiliser des oxydes, des oxydes mixtes, des hydroxydes, des sels divers tels que des oxyhalogénures ainsi que des anhydrides, hétéropolyacides et acides et les sels correspondants. Comme composés organiques, on peut utiliser les esters des acides minéraux dérivés du sélénium, les acides organiques du sélénium ainsi que les composés organométalliques du sélénium tels que le sélénium carbonylé.

Les oxydes, acides (et esters correspondantes), anhydrides et acides organiques du sélénium ont donné de bons résultats. Les meilleurs résultats ont été obtenus avec le dioxyde de sélénium et l'acide sélénieux.

Les composés du sélénium sont en général mis en oeuvre en quantités supérieures à 0,01, le plus

souvent supérieures à 0,05 et de préférence supérieures à 0,1 g par litre de mélange réactionnel. Ces quantités ne dépassent en général pas 25 et de préférence pas 10 g par litre.

Les composés du sélénium peuvent être ajoutés au mélange réactionnel à l'état pur ou sous la forme d'une solution dans un des constituants du mélange réactionnel.

Les bases organiques azotées mises en oeuvre dans le procédé selon l'invention ont un pKa dans l'eau qui ne dépasse pas 8 et de préférence pas 7,5. On évite ainsi notamment une inactivation du catalyseur et une inhibition de la réaction. En général, le pKa de la base mise en oeuvre est d'au moins 2 et de préférence d'au moins 2,5. Des bases à pKa inférieur peuvent néanmoins être utilisées mais, dans ce cas, elles doivent être mises en oeuvre en quantités plus importantes. Les meilleurs résultats ont été obtenus avec les bases dont le pKa est compris entre 2,5 et 7,5. La base mise en oeuvre doit en outre être de préférence inerte vis-à-vis du peroxyde d'hydrogène dans les conditions de réaction. On peut utiliser une seule base ou un mélange de bases.

Des bases convenant bien sont celles qui sont inertes vis-à-vis du peroxyde d'hydrogène dans les conditions de réaction et qui contiennent dans leur structure un cycle à caractère aromatique comme la pyridine, ses dérivés halogénés, ses dérivés aminés tels que la 2-aminopyridine, la 3-aminopyridine et la 2,5-diaminopyridine, ses dérivés alcoxylés tels que la 3-méthoxypyridine et la 4-méthoxypyridine et ses dérivés alkylés tels que les picolines, les lutidines (-2,3, -2,4, -2,5, -2,6, -3,4, -3,5, -3,6), les collidines, les éthylpyridines et les propylpyridines, les dérivés N-alkylés de l'aniline tels que la N-méthylaniline le NN-diméthylaniline, la N-éthylaniline et la NN-diéthylaniline, les N-méthyl toluidines, les NN-diméthyltoluidines, les N-éthyltoluidines la N-allylamine, la quinoléine, et l'isoquinoléine et leurs dérivés tels que la 1-aminoisoquinoléine, la 3-aminoquinoléine, les méthylquinoléines, les diméthylquinoléines, et la 6-méthoxyquinoléine, l'imidazole et ses dérivés tels que le 1-méthylimidazole ainsi que le benzimidazole et ses dérivés tels que le 2-méthylbenzimidazole, le 2-éthylbenzimidazole et le 2-phénylbenzimidazole. La pyridine, la chloropyridine, la quinoléine et l'isoquinoléine ainsi que leurs dérivés substitués par un ou plusieurs groupes alkyles contenant 1 ou 2 atomes de carbone par groupe alkyle, conviennent tout particulièrement bien. De bons résultats ont été obtenus avec la pyridine, la chloropyridine, la collidine, la quinoléine, l'isoquinoléine et les lutidines. On peut mettre en oeuvre une seule base ou plusieurs.

Les bases peuvent être mises en oeuvre en quantités variables. En général, on ajoute au mélange réactionnel au moins 0,01 mole de base par atome-gramme de métal du catalyseur. En général, la quantité de base mise en oeuvre ne dépasse pas 20 moles et le plus souvent pas 10 moles par atome-gramme de métal. De bons résultats ont été obtenus avec des quantités comprises entre 0,1 et 10 et de préférence 0,3 et 6 moles de base par atome-gramme de métal du catalyseur. La quantité totale de base présente dans le mélange réactionnel ne dépasse en général pas 4% de son poids.

Les composés contenant une double liaison éthylénique utilisables comme réactif dans le procédé selon l'invention peuvent être de nature très diverses. Ces oléfines contiennent une ou plusieurs doubles liaisons éthyléniques >C=C<. Elles peuvent être aliphatiques, alicycliques ou aromatiques. Elles peuvent éventuellement contenir un hétéroatome ou plus dans leur chaîne principale, en général choisi parmi les atomes d'azote, de soufre ou d'oxygène. Elles peuvent être substituées par divers atomes ou groupements stables dans le mélange réactionnel tels que des halogènes et plus particulièrement des atomes de chlore, de fluor ou de brome, des groupements hydroxy, alkoxy, nitro, amino, carbonyles, nitriles, acides, esters, amides ou encore par des groupements aromatiques, aliphatiques ou alicycliques éventuellement eux-mêmes substitués. En général, elles contiennent de 2 à 40 atomes de carbone.

Le procédé selon l'invention est appliqué en général à des oléfines répondant à la formule générale

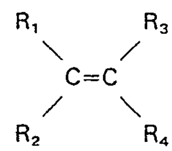

où $R_1$, $R_2$, $R_3$ et $R_4$ représentent des atomes ou des groupements identiques ou différents choisis parmi l'hydrogène, les halogènes et plus particulièrement le chlore, le fluor ou le brome, les groupements alkyles, cycloalkyles ou aryles substitués ou non et contenant ou non un ou plusieurs hétéroatomes dans leur structure, et les groupements éthers, acides, esters, nitriles ou amides ou bien où $R_1$ et $R_2$ ou $R_3$ et $R_4$ représentent deux à deux une chaîne alkylée substituée ou non et contenant ou non un ou plusieurs hétéroatomes, ou bien où $R_1$ et $R_3$ ou $R_2$ et $R_4$ représentent deux à deux une chaîne alkylée substituée ou non et contenant ou non un ou plusieurs hétéroatomes.

A titre d'exemples de telles oléfines on peut citer la propylène, le butène-1, le butène-2, l'isobutène, le butadiène, les pentènes et notamment le pentène-1, le 2 - méthyl - 1 - butène, le 3 - méthyl - 1 - butène, le 2 - méthyl - 2 - butène, le pipérylène, les hexènes -1, -2 et -3, les hexadiènes, le 2,3 - diméthyl - 2 - butène, l'heptène-1, le 3 - éthylpentène - 2, l'octène-1, le diisobutylène, les 2,4,4 - triméthylpentène - 1 et -2, les octadiènes, le nonène-1, le décène-1, l'undécène-1, le dodécène-1, le tridécène-1, le tétrádècène-1, le pentadécène-1, l'hexadécène-1, l'heptadécène-1, l'ocytadécène-1, le nonadécène-1, l'eicosène-1, les trimères et tétramères du

propylène, les polybutadiènes, l'isoprène et les terpènes tels que les terpinènes, le limonène, le terpinolène, le sabinène, le pinène, le camphène, le myrcène, le cadinène, le cédrène, le santalène, le calarène, le colophène et les polyterpènes ainsi que leurs dérivés tels que le géraniol, le linalol et l'acétate de linalyle, le méthylènecyclopropane, le cyclopentène, et ses dérivés substitués par des groupes alkyles ou aryles, le cyclopentadiène, le cyclohexène et ses dérivés substitués par des groupes alkyles et aryles, le méthylènecyclopentane, le cyclohexadiène, le méthylènecyclohexane, le norbornène, le cycloheptène, le vinylcyclohexane, le vinylcyclohexène, le styrène, le cyclooctène, les cyclooctadiènes, la vinylnorbornène, l'indène substitué ou non, le tétrahydroindène, l'alpha-méthylstyrène, et les alpha-alkylstyrènes éventuellement substitués sur le noyau aromatique le dicyclopentadiène, le divinylbenzène, les dihydronaphtalènes substitués ou non, le cyclododécène, le cyclododécatriène, le stilbène, le 2,3 - diphényl - 2 - butène, le diphénylbutadiène, la vitamine A, le béta-carotène, le fluorure de vinylidène, le chlorure et le bromure d'allyle, les trichloropropylènes, le chlorure de crotyle, le chlorure de méthallyle, les chlorobutènes, les dichlorobutènes, les dibromobutadiènes, les dichlorobutadiènes, l'hexafluorobutadiène, l'alcool allylique et l'alcool méthallylique ainsi que leurs dérivés alkylés ou arylés, le 1 - butène - 4 - ol et ses dérivés alkylés en 2 et 4, le 2 - butène - 1 - ol, le 2 - butène - 1,4 - diol, les cyclopentènediols, les 4-penténols, le 2 - méthylpentène - 2 - ol - 1, le 1,2 - dihydroxy - 4 - vinylbenzène, le béta-chlorostyrène et les alpha - alkyl - béta - chlorostyrènes éventuellement substitués sur le noyau aromatique, le 2,7 - octadiénol - 1, le cyclohexylcarbinol, le tridécène - 2 - ol - 1, les 2- et 3-alkoxy- et aryloxy-propènes et leurs dérivés substitués, les stéroïdes insaturés, l'éthoxyéthylène, le diallyléther et ses dérivés substitués, l'isoeugénol, l'anithole, l'isosafrole, le dihydrofurane et ses dérivés alkylés, le benzofurane et ses dérives substitués, les acides carboxyliques insaturés de tous types tels que l'acide acrylique, l'acide méthacrylique, les acides alpha- et béta-cyanoacryliques et leurs dérivés, l'acide crotonique, l'acide maléique et ses dérivés alkylés, l'acide vinylacétique et les acides gras insaturés parmi lesquels figurent plus particulièrement les acides oléique, linoléique, palmitoléique, linolénique, vaccinique, gadoléique, ricinoléique et éléostéarique et les graisses et huiles naturelles qui en contiennent ainsi que les esters de tous des acides insaturés tels que les acrylates et méthacrylates d'alkyle, le maléate de diallyle, le 7 - hydroxy - 5 - hepténoate de méthyle, l'oléate de méthyle et les esters d'alcools insaturés tels que le carbonate d'allyle, le phtalate de diallyle, l'acétate d'allyle.

Le procédé selon l'invention convient particulièrement bien pour époxyder les oléfines acycliques ou cycliques no contenant pas plus de 6 atomes de carbone par cycle. Il convient tout particulièrement bien pour époxyder les oléfines contenant de 2 à 20 atomes de carbone et plus spécialement les oléfines internes (c'est-à-dire les composés cycliques qui comportent au moins une double liaison éthylénique dans un cycle) éventuellement substituées et les alkènes substitués.

De bons résultats ont été obtenus lors de l'époxydation du cyclohexène, du 2-méthylbutène-2, du styrène, du chlorure d'allyle, de l'alpha-pinène, de l'alcool allylique et du dihydrofurane.

Le peroxyde d'hydrogène peut être mis en oeuvre sous la forme d'une solution aqueuse ou sous la forme d'une solution organique. Pour des raisons économiques on le met en général en oeuvre sous la forme d'une solution aqueuse. Des solutions contenant au moins 20% et de préférence au moins 30% de peroxyde d'hydrogène conviennent bien. Pour des raisons de disponibilité, les solutions mises en oeuvre no contiennent en général pas plus de 95% et le plus souvent pas plus de 90% de peroxyde d'hydrogène.

On peut opérer avec des rapports molaires oléfine sur peroxyde d'hydrogène dans le mélange réactionnel qui peuvent varier dans de larges limites. En général, on préfère utiliser des rapports molaires d'au moins 0,9 pour assurer une bonne sélectivité. Le plus souvent, ce rapport molaire est supérieur à 1,5. En général, le rapport molaire est inférieur à 10 et de préférence à 5 pour des raisons économiques, de manière à ne pas devoir recycler de trop grandes quantités d'oléfines non transformées. De bons résultats ont été obtenus avec des rapports molaires voisins de 3.

Les conditions de réaction sont choisies de manière à obtenir un mélange homogène. Pour ce faire, il est avantageux de mettre en oeuvre un solvant organique. La mise en oeuvre d'un solvant organique est moins avantageuse lorsque l'oléfins est capable de solubiliser le peroxyde d'hydrogène, l'époxyde et le catalyseur aux concentrations utilisées. Il en est souvent ainsi lorsque l'oléfine comporte dans sa structure des fonctions éthers ou des groupes hydroxyles ou carboxyles comme c'est le cas per exemple des alcools insaturés tels que l'alcool allylique, des esters insaturés et des éthers acycliques ou cycliques insaturés tels que le dihydrofurane. Dans ce cas, on peut être amené à mettre en oeuvre un large excès d'oléfine à époxyder, de sorte que le rapport molaire oléfine sur peroxyde d'hydrogène peut atteindre 30.

Lorsqu'on met en oeuvre un solvant organique, on le choisit de préférence tel qu'il solubilise l'oléfine, le peroxyde d'hydrogène, l'époxyde et le catalyseur aux concentrations utilisées. Le solvant doit être en outre inerte vis-à-vis réactifs mis en oeuvre dans les conditions de réaction. On peut utiliser un solvant unique ou un mélange de solvants. Le solvant est choisi en général parmi les alcools, éthers, cycliques et esters contenant de 2 à 12 atomes de carbone et de préférence de 3

à 8 atomes de carbone. On utilise de préférence des alcools primaires, secondaires ou tertiaires. Le plus souvent, on utilise un solvant choisi parmi l'éthanol, le n-propanol, l'isopropanol, le butanol-1, le butanol-2, le tert-butanol, l'alcool amylique, l'alcool isoamylique, l'alcool tert-amylique, le cyclohexanol, l'alcool benzylique, l'heptanol-1, l'hexanol. De bons résultats ont été obtenus avec l'alcool benzylique et le n-butanol.

Le solvant peut être mis en oeuvre en quantités variables. En général, le mélange réactionnel contient au moins 20% en poids de solvant pour éviter la formation de deux phases liquides, soit avant la réaction, soit en cours de réaction. En général, la quantité de solvant ne dépasse pas 90% du mélange de manière à simplifier les opérations de séparation et à ne pas avoir de réduction trop importante de vitesses de réaction par dilution. Le mélange réactionnel contient de préférence de 30 à 80% en poids de solvant. De bons résultats ont été obtenus lorsque le rapport molaire oléfine sur solvant est voisin de 1.

L'eau contenue dans le mélange réactionnel en petites quantités est en général l'eau formée par la réaction ou l'eau introduite avec les réactifs. De préférence, on maintient la quantité d'eau présente à moins de 5% du poids du mélange réactionnel.

On peut également ajouter au mélange réactionnel d'autres additifs tels que des agents de stabilisation du peroxyde d'hydrogène, des inhibiteurs de polymérisation ou éventuellement des dérivés minéraux ou organiques susceptibles de fixer l'eau du milieu réactionnel. Ces additifs éventuels sont en général présents à raison de moins de 3% du poids du mélange réactionnel.

La température et la pression auxquelles on opère la réaction peuvent varier dans de très larges limites. Elles sont choisies en fonction de la nature de l'oléfine à époxyder et de manière à ne pas dépasser la température de décomposition du mélange réactionnel. La température est habituellement inférieure à 150°C et le plus souvent comprise entre 0 et 120°C. De bons résultats ont été obtenus à des températures comprises entre 40 et 100°C. La pression de réaction peut être inférieure, égale ou supérieure à la pression atmosphérique. La pression est en général inférieure à 5 atmosphères. De bons résultats ont été obtenus en utilisant des pressions de 0,05 à 3 atmosphères. En général, on règle la température et la pression de manière à assurer l'ébullition du mélange réactionnel.

La durée de réaction dépend de la nature de l'oléfine à époxyder ainsi que du catalyseur, du solvant et de la base mise en oeuvre. Elle peut aller de 1 minute à 50 heures.

Le procédé selon l'invention peut être mis en oeuvre en continu ou en discontinu dans un réacteur unique ou dans une série de réacteurs en parallèle ou en série. Pour réaliser le procédé selon l'invention, on peut utiliser n'importe quel appareillage convenant pour les mélanges réactionnels liquides.

Les catalyseurs, les bases et les réactifs peuvent être introduits de diverses manières connues en elles-mêmes. On peut ainsi procéder à une introduction unique, une introduction continue ou une introduction étagée du catalyseur, de la base, de l'oléfine ou du peroxyde d'hydrogène.

Un mode de réalisation particulier du procédé selon l'invention consiste à maintenir le mélange réactionnel substantiellement anhydre. Dans ce cas, on maintient dans le mélange une concentration en eau inférieure de préférence à 2% de son poids. De très bons résultats ont été obtenus lorsque le mélange réactionnel contient moins de 1% en poids d'eau.

Pour maintenir le mélange à l'état substantiellement anhydre, on élimine en continu l'eau venant à s'y trouver. Pour ce faire, on peut utiliser diverses techniques. En général, l'eau présente dans le mélange réactionnel est éliminée par des procédés de vaporisation tels que la distillation, la distillation azéotropique ou l'entraînement au moyen d'un gaz inerte.

Si l'eau forme avec un des constituants du mélange tel que le solvant, un azéotrope à minimum dont la température d'ébullition est plus faible que celle des autres constituants du mélange et des autres azéotropes éventuels qui pourraient se former, on élimine en général l'eau par distillation azéotropique.

On peut également ajouter au mélange réactionnel au moins un agent de distillation azéotropique de nature différente de celle du solvant de réaction. Cet agent est choisi parmi ceux capables de former avec l'eau un azéotrope à minimum dont la température d'ébullition est plus faible que celle des autres constituants du mélange et des autres azéotropes éventuels qui pourraient se former. Il est choisi inerte vis-à-vis des autres constituants du mélange dans les conditions de réaction. De plus il est choisi de manière à ne pas perturber l'homogénéité du mélange de réaction. Le plus souvent, il est choisi parmi les hydrocarbures chlorés et les hydrocarbures aromatiques. Les hydrocarbures chlorés contenant de 1 à 6 atomes de carbone et les hydrocarbures aromatiques éventuellement substitués par des groupes alkyles ou des halogènes contenant de 6 à 12 atomes de carbone conviennent bien. De bons résultats ont été obtenus avec le chlorure de méthylène, le chloroform, et 1,2-dichloroéthane, les 1,2-dichloropropanes et le benzène.

L'agent d'entraînement est mis en oeuvre en quantités variables, les doses étant choisies de manière à maintenir l'homogénéité du mélange de réaction. En général, il est mis en oeuvre à des doses ne dépassant pas 50% et les plus souvent 30% du poids du mélange réactionnel. Quand on a recours à un agent de distillation azéotropique on le met oeuvre à des doses en général d'au moins 1% et le plus souvent 3% du poids du mélange réactionnel.

Cette technique peut être avantageusement utilisée lorsqu'on ne met pas en oeuvre un solvant de réaction ou lorsque l'eau ne forme pas avec le solvant de réaction un azéotrope à minimum dont la température d'ébullition est plus faible que celle des autres constituants du mélange et des autres azéotropes éventuels qui pourraient se former.

L'élimination de l'eau par distillation azéotropique convient tout particulièrement bien lorsque l'azéotrope ainsi formé est un azéotrope hétérogène, car il est alors possible de recycler la phase organique dans le mélange réactionnel, après séparation de la phase aqueuse du distillat.

Lorsque la température d'ébullition de l'eau est plus faible que celle des autres constituants du mélange réactionnel et des azéotropes éventuels qui pourraient se former, on utilise le plus souvent un procédé de distillation ou un procédé d'entraînement de l'eau par passage en continu d'un gaz inerte dans le mélange réactionnel. Cette dernière technique est en général utilisée lorsqu'on veut éviter de porter à l'ébullition des mélanges susceptibles de se décomposer à leur température d'ébullition.

Selon un autre mode particulier de réalisation du procédé selon l'invention, on fait réagir l'oléfine avec le peroxyde d'hydrogène sans éliminer en continu l'eau se trouvant dans le mélange réactionnel. Dans ce cas, on peut avantageusement chauffer à reflux le mélange réactionnel pour éliminer la chaleur de réaction.

Après réaction, le mélange peut être soumis à diverses techniques de séparation telles que la distillation et la décantation pour recueillir l'époxyde et les réactifs non transformés que l'on peut avantageusement recycler au procédé.

Le procédé selon l'invention peut être réalisé en continu dans un appareil tel que celui représenté schématiquement à la figure unique du dessin en annexe qui se rapporte à un mode de réalisation pratique particulier.

Dans un réacteur 1 surmonté d'une colonne de distillation 2, on introduit par la voie 3, une solution concentrée de peroxyde d'hydrogène et par le voie 4 la solution organique contenant le catalyseur, la base et l'oléfine. L'oléfine, le catalyseur et la base sont introduits par la voie 5.

En cours de réaction l'azéotrope eau-solvant quitte la colonne de distillation 2 par la voie 9, est condensé dans le condenseur 10, et est envoyé par le voie 11 au décanteur 12. Lorsque le solvant a une densité inférieure à celle de l'eau, on la recueille en tête du décanteur par la voie 13 tandis que l'eau est recueillie au pied du décanteur par la voie 14; dans le cas contraire, les prélèvements sont inversés. Le solvant est recyclé à la colonne de distillation par la voie 15 où il constitue le reflux. Dans certains cas, on peut envoyer une partie du solvant par la voie 16 dans le mélangeur 6 qui est alimenté par la voie 5 an oléfine, en base et en catalyseur.

On soutire en continue par la voie 7 une partie du mélange réactionnel que l'on soumet à des séparations successives pour obtenir d'une part l'oléfine non transformée que l'on renvoie en 6 et d'autre part l'epoxyde qui constitue la production.

Afin d'illustrer l'invention, sans pour autant en limiter la portée, on donne ci-après des exemples de fabrication d'époxydes conformes à l'invention (exemples 1, 4, 5, 7, 8, 9 à 13).

Les exemples 2R, 3R, 6R, sont donnés à titre de comparaison.

Exemple 1 (selon l'invention)—Epoxydation du cyclohexène

Dans un réacteur en verre à double enveloppe, chauffé par circulation d'huile, muni d'une agitation magnétique et d'un réfrigérant à reflux avec décanteur florentin tel que représenté à la figure 1, on introduit successivement 100 ml d'alcool benzylique (966 mmoles), 80 ml de cyclohexène (790 mmoles), 0,45 ml de pyridine (5,6 mmoles) et 200 mg de $SeO_2$ (1,8 mmoles). Le florentin contient lui-même 20 ml de cyclohexène (198 mmoles). Après avoir porté le mélange réactionnel à la température de 75°C, on introduit en 10 minutes 10 ml de $H_2O_2$ à 84% (340 mmoles). Après avoir encore laisse réagir 10 minutes, on dose l'oxyde de cyclohexène formé par chromatographie en phase gazeuse: 287 mmoles.

La sélectivité par rapport à $H_2O_2$ consommé est de 86% (taux de transformation: 98%).

La productivité en oxyde de cyclohexène est de 469 g/h.litre de mélange réactionnel.

Exemple 2R (de référence)—Epoxydation du cyclohexène

Dans un réacteur muni d'une agitation magnétique et d'un réfrigérant à reflux muni d'un florentin tel que celui représenté à la figure 1, on introduit 41 g d'alcool amylique, 39 mg de dioxyde de sélénium (0,35 mmole), 0,3 g de phosphate disodique $Na_2HPO_4$ et 41 g de cyclohexène (499 mmoles). Le florentin contient lui-même 20 ml de cyclohexène (198 mmoles). On porte ce mélange à la température de 81°C et on introduit en 12 minutes 5 ml d'une solution aqueuse de peroxyde d'hydrogène à 30% en poids (49,5 mmoles). On laisse réagir pendant deux heures en éliminant continuellement l'eau par distillation azéotropique. La température se maintient entre 80°C et 90°C.

L'oxyde de cyclohexène formé est dosé par chromatographie en phase gazeuse: 7,6 mmole.

La sélectivité par rapport à $H_2O_2$ consommé est de 28% (taux de transformation 55%).

La productivité en oxyde de cyclohexène est de 3 g/h.litre de mélange réactionnel.

Exemple 3R (de référence)—Epoxydation du cyclohexène

Dans un réacteur muni d'une agitation magnétique et d'un réfrigérant à reflux muni d'un florentin, on introduit 41 g d'alcool amylique, 0,05 g d'oxyde de molybdène de formule $MoO_3$ (0,34 mmole), 0,3 g de phosphate disodique $Na_2HPO_4$ et 41 g de cyclohexène (499 mmoles). Le florentin contient lui-même 20 ml de cyclohexène (198 mmoles). On porte ce mélange à la température de 81°C et on introduit en 12 minutes 5 ml d'une solution aqueuse de peroxyde d'hydrogène à 84% en poids (168 mmoles). On laisse réagir pendant une heure en éliminant continuellement

l'eau par distillation azéotropique. La température se maintient entre 80°C et 90°C.

L'oxyde de cyclohexène formé est dosé par chromatographie en phase gazeuse: 3,9 mmoles.

La sélectivité par rapport à $H_2O_2$ consommé est de 15% (taux de transformation 41%).

La productivité en oxyde de cyclohexène est de 3 g/h.litre du mélange réactionnel.

Exemple 4 (selon l'invention)—Epoxydation du 2-méthyl-3-butène

Dans un réacteur en verre à double enveloppe, chauffé par circulation d'huile, muni d'une agitation magnétique et d'un réfrigérant à reflux avec florentin tel que représenté à la figure 1, on introduit successivement 100 ml d'alcool benzylique, 100 ml de 2 - méthyl - 2 - butène (944 mmoles), 0,8 ml de quinoline (6,78 mmoles), 250 mg de $SeO_2$ (2,25 mmoles) et 5 ml de $H_2O_2$ à 84% (170 mmoles). On chauffe à reflux et l'on ajoute 5 ml de $H_2O_2$ à 84% (170 mmoles) en 5 minutes. On laisse encore réagir 15 minutes puis l'on dose l'oxyde d'isoamylène formé: 272 mmoles.

La sélectivité par rapport à $H_2O_2$ consommé est de 84% (taux de transformation 95%).

La productivité en oxyde d'isoamylène est de 351 g/h.litre de mélange réactionnel.

Exemple 5 (selon l'invention)—Epoxydation du 2-méthyl-2-butène

Dans un réacteur en verre à double enveloppe, chauffé par circulation d'huile, muni d'une agitation magnétique et d'un réfrigérant à reflux sans décanteur florentin, on introduit successivement 100 ml d'alcool benzylique, 100 ml de 2 - méthyl - 2 - butène (944 mmoles), 0,8 ml de quinoléine (6,78 mmoles), 250 mg de $SeO_2$ (2,25 mmoles) et 5 ml de $H_2O_2$ à 84% (170 mmoles). On chauffe à reflux et on ajoute 5 ml de $H_2O_2$ à 84% (170 mmoles) en 5 minutes. On laisse encore réagir 15 minutes puis l'on dose l'oxyde d'isoamylène formé: 269 mmoles.

La sélectivité par rapport à $H_2O_2$ consommé est de 92% (taux de transformation: 87%).

La productivité en oxyde d'isoamylène est de 347 g/h.litre de mélange réactionnel.

Exemple 6R (de référence)—Epoxydation du 2-méthyl-2-butène

Dans un réacteur en verre à double enveloppe chauffé par circulation d'huile, muni d'une agitation magnétique et d'un réfrigérant à reflux avec florentin tel que représenté à la figure 1, on introduit successivement 100 ml de n-butanol (1093 mmoles), 100 ml de 2 - méthyl - 2 - butène (944 mmoles), 1,09 ml de pyridine (13 mmoles), 324 mg de $MoO_3$ (2,25 mmoles) et 10 ml de $H_2O_2$ à 84% (326 mmoles). On chauffe alors à reflux pendant 1 h puis l'on dose l'oxyde d'isoamylène formé par chromatographie en phase gazeuse: 39 mmoles.

La sélectivité par rapport à $H_2O_2$ consommé est de 66% (taux de transformation: 18%).

La productivité en oxyde d'isoamylène est de 17 g/h.litre de mélange réactionnel.

Exemple 7 (selon l'invention)—Epoxydation de 2-méthyl-2-butène

Dans un réacteur en verre à double enveloppe, chauffé par circulation d'huile, muni d'une agitation magnétique et d'un réfrigérant à reflux sans décanteur florentin, on introduit successivement 100 ml d'alcool isopropylique, 100 ml de 2 - méthyl - 2 - butène (944 mmoles), 0,315 ml de triéthylamine (2,26 mmoles), 290 mg d'acide sélénieux (2,25 mmoles) et 5 ml de $H_2O_2$ à 84% (170 mmoles). On chauffe à reflux (41 à 42°C) et l'on ajoute 5 ml de $H_2O_2$ à 84% (170 mmoles) en 5 minutes. Après 1 h de réaction, on dose l'oxyde d'isoamylène formé: 73 mmoles.

La sélectivité par rapport à $H_2O_2$ consommé est de 100% (taux de transformation: 22%).

La productivité en oxyde d'isoamylène est de 31 g/h.litre de mélange réactionnel.

Exemple 8 (selon l'invention)—Epoxydation du 2-méthyl-2-butène

Dans un réacteur analogue à celui de l'exemple 1, mais muni d'une petite colonne de Vigreux, on introduit successivement 78 ml de n-butanol (852 mmoles), 130 ml de 2 - méthyl - 2 - butène (1202 mmoles), 1,87 ml de quinoléine (15,9 mmoles) et 208 mg de $SeO_2$ (1,9 mmoles). Après avoir porté le mélange réactionnel à la température de 43°C, on introduit en 45 minutes 20 ml de $H_2O_2$ environ 50% en poids (354 mmoles). Après avoir encore laissé réagir 26 minutes, on dose l'oxyde d'isoamylène formé par chromatographie en phase gazeuse: 291 mmoles.

La sélectivité par rapport à $H_2O_2$ consommé est de 91% (taux de transformation: 90%).

La productivité en oxyde d'isoamylène est de 102 g/h.litre de mélange réactionnel.

Exemple 9 (selon l'invention)—Epoxydation de l'alpha-pinène

Dans un réacteur analogue à celui de l'exemple 1, on introduit successivement 48 ml de n-butanol (525 mmoles), 152 ml d'alpha-pinène (975 mmoles), 1,15 ml de pyridine (14 mmoles) et 807 mg de $SeO_2$ (7,3 mmoles). Le florentin contient lui-même 25 ml de n-butanol (273 mmoles). Après avoir porté le mélange réactionnel à la température de 50°C, on introduit en 25 minutes 10 ml de $H_2O_2$ à 84% (340 mmoles). Après avoir encore laissé réagir 39 minutes, on dose l'oxyde d'alpha-pinène formé par chromatographie en phase gazeuse: 292 mmoles.

La sélectivité par rapport à $H_2O_2$ consommé est de 88% (taux de transformation: 98%).

La productivité en oxyde d'alpha-pinène est de 208 g/h.litre de mélange réactionnel.

Exemple 10 (selon l'invention)—Epoxydation du styrène

Dans un réacteur analogue à celui de l'exemple 1, mais muni d'une petite colonne de Vigreux, on introduit successivement 55 ml de n-butanol (601 mmoles), 145 ml de styrène (1266 mmoles), 1,12 ml de pyridine (14 mmoles) et 572 mg de $SeO_2$ (5,2 mmoles). Le florentin contient lui-même 25

ml de n-butanol (273 mmoles). Après avoir porté le mélange réactionnel à la température de 68°C, on introduit en 24 minutes 10 ml de $H_2O_2$ à 84% (340 mmoles). Après avoir encore laissé réagir 47 minutes, on dose l'oxyde de styrène forme par chromatographie en phase gazeuse: 190 mmoles.

La sélectivité par rapport à $H_2O_2$ consommé est de 63% (taux de transformation: 89%).

La productivité en oxyde de styrène est de 120 g/h.litre de mélange réactionnel.

Exemple 11 (selon l'invention)—Epoxydation de l'alcool allylique

Dans un réacteur en verre à double enveloppe, chauffé par circulation d'huile, muni d'une agitation magnétique et d'un réfrigérant à reflux avec décanteur florentin tel que représenté à la figure 1, on introduit successivement 100 ml d'alcool allylique (1470 mmoles), 0,25 ml de pyridine (3,1 mmoles), 450 mg de $SeO_2$ (4,1 mmoles) et 10 ml de $H_2O_2$ à 84% (340 mmoles). On porte le mélange réactionnel à la température de 70°C sous une pression de 15 000 Pa. On laisse réagir pendant une heure en soutirant l'azéotrope oléfine-eau condensé dans le florentin et en compensant ce volume par un ajout continu d'alcool allylique (75 ml en une heure). On dose le glycidol formé par chromatographie en phase gazeuse: 284 mmoles. La sélectivité par rapport à $H_2O_2$ consommé est de 95% (taux de transformation 82%).

La productivité en glycidol est de 210 g/h.litre de mélange réactionnel.

Exemple 12 (selon l'invention)—Epoxydation du 2,5-dihydrofurane

Dans un réacteur en verre à double enveloppe, chauffé par circulation d'huile, muni d'un agitateur magnétique et d'un réfrigérant à reflux avec décanteur florentin tel que représenté à la figure 1, on introduit successivement 100 ml de 2,5-dihydrofurane (1263 mmoles), 0,38 ml de 2-chloropyridine (4,0 mmoles), 450 mg de $SeO_2$ (4,1 mmoles) et 10 ml de $H_2O_2$ à 84% (340 moles). Le florentin contient lui-même 25 ml de $CHCl_3$ (312 mmoles). On porte le mélange réactionnel à la température de 64°C et on laisse réagir pendant 1 h.

Le mélange recueilli dans le florentin comporte une phase aqueuse que l'on rejette et une phase organique riche en chloroforme et contenant en outre du 2,5-dihydrofurane que l'on recycle au réacteur. On dose le 3,4 - époxy - 2,5 - dihydrofurane formé par chromatographie en phase vapeur: 203 mmoles.

La sélectivité par rapport à $H_2O_2$ consommé est de 76% (taux de transformation 79%).

La productivité en époxyde est de 175 g/h.litre de mélange réactionnel.

Exemple 13 (selon l'invention)—Epoxydation de 2,5-dihydrofurane

Dans une ampoule à robinet, on introduit 100 ml de 2,5-dihydrofurane (1263 mmoles) contenant 7% d'$H_2O$ et 50 ml de benzène (559 mmoles).

Après décantation, on élimine la phase aqueuse (3,5 ml). La phase organique est alors transvasée dans un réacteur à double enveloppe, chauffé par circulation d'huile, muni d'une agitation magnétique et d'un réfrigérant à reflux avec décanteur florentin tel que celui représenté à la figure 1. On introduit successivement dans le réacteur 0,15 ml de 2-chloropyridine (1,6 mmoles), 450 mg de $SeO_2$ (4,1 mmoles) et 2 ml de $H_2O_2$ à 84% (68 mmoles). On porte le mélange réactionnel à la température de 70°C et on introduit en 10 minutes 8 ml d'$H_2O_2$ à 84% (272 mmoles). On laisse réagir pendant 50 minutes en éliminant continuellement l'eau par distillation azéotropique. On dose le 3,4 - époxy - 2,5 - dihydrofurane formé par chromatographie en phase vapeur: 253 mmoles.

La sélectivité par rapport à $H_2O_2$ consommé est de 78% (taux de transformation 95%).

La productivité en époxyde est de 145 g/h.litre de mélange réactionnel.

## Revendications

1. Procédé pour la fabrication d'époxydes selon lequel on fait réagir du peroxyde d'hydrogène avec un composé contenant une double liaison éthylénique dans un mélange réactionnel homogène liquide contenant un catalyseur et une base, caractérisé en ce que l'on maintient la concentration en eau du mélange réactionnel à moins de 10% en poids, on utilise une base choisie parmi les bases organiques azotées ayant un pKa ne dépassant pas 8 et on utilise un catalyseur choisi parmi les composés du sélénium.

2. Procédé selon la revendication 1, caractérisé en ce qu'on choisit le catalyseur parmi le dioxyde de sélénium et l'acide sélénieux.

3. Procédé selon l'une quelconque des revendications 1 et 2, caractérisé en ce que le catalyseur est mis en oeuvre en quantités comprises entre 0,05 et 25 g par litre de mélange réactionnel.

4. Procédé selon l'une quelconque des revendications 1 à 3, caractérisé en ce qu'on choisit la base parmi les bases inertes vis-à-vis du peroxyde d'hydrogène contenant dans leur structure un cycle à caractère aromatique.

5. Procédé selon la revendication 4, caractérisé en ce qu'on choisit la base parmi la pyridine, la chloropyridine, la quinoléine, l'isoquinoléine et leurs dérivés substitués par un ou plusieurs groupes alkyles contenant de 1 à 2 atomes de carbone par groupe alkyle.

6. Procédé selon l'une quelconque des revendications 1 à 5, caractérisé en ce que la base est mise en oeuvre en quantités comprises entre 0,1 et 10 moles par atome-gramme de métal du catalyseur.

7. Procédé selon l'une quelconque des revendications 1 à 6, caractérisé en ce qu'on choisit le composé contenant une double liaison éthylénique parmi les oléfines contenant de 2 à 20

atomes de carbone, acycliques ou cycliques ne contenant pas plus de 6 atomes de carbone par cycle.

8. Procédé selon l'une quelconque des revendications 1 à 7, caractérisé en ce que le composé contenant une double liaison éthylénique est mis en oeuvre en quantités comprises entre 1,5 et 5 moles par mole de peroxyde d'hydrogène.

9. Procédé selon l'une quelconque des revendications 1 à 8, caractérisé en ce qu'on opère en présence d'un solvant organique inerte choisi parmi les alcools primaires, secondaires ou tertiaires contenant de 3 à 8 atomes de carbone.

**Patentansprüche**

1. Verfahren zur Herstellung von Epoxiden, wobei man Wasserstoffperoxid mit einer Verbindung, die eine ethylenartige Doppelbindung enthält, in einer homogenen flüssigen Reaktionsmischung, die einen Katalysator und eine Base enthält, umsetzt, dadurch gekennzeichnet, daß man die Wasserkonzentration die Reaktionsmischung unter 10 Gew.-% hält, eine Base, die ausgewählt wird unter organischen Stickstoffhaltigen Basen mit einem pKa, der 8 nicht überschreitet, verwendet, und einen Katalysator, der ausgewählt wird unter Selenverbindungen, verwendet.

2. Verfahren nach Anspruch 1, dadurch gekennzeichnet, daß man den Katalysator auswählt unter Selendioxid und seleniger Säure.

3. Verfahren nach einem der Ansprüche 1 und 2, dadurch gekennzeichnet, daß der Katalysator in Mengen zwischen 0,05 und 25 g/l der Reaktionsmischung verwendet wird.

4. Verfahren nach einem der Ansprüche 1 bis 3, dadurch gekennzeichnet, daß man die Base unter den gegenüber Wasserstoffperoxid inerten Basen auswählt, die in ihrer Struktur einen Ring mit aromatischem Charakter enthalten.

5. Verfahren nach Anspruch 4, dadurch gekennzeichnet, daß man die Base auswählt unter Pyridin, Chlorpyridin, Chinolin, Isochinolin und deren Derivate, die durch eine oder mehrere Alkylgruppen mit 1 bis 2 Kohlenstoffatomen pro Alkylgruppe substituiert sind.

6. Verfahren nach einem der Ansprüche 1 bis 5, dadurch gekennzeichnet, daß die Base in Mengen zwischen 0,1 und 10 mol pro Grammatom Metall des Katalysators eingesetzt wird.

7. Verfahren nach einem der Ansprüche 1 bis 6, dadurch gekennzeichnet, daß man die eine ethylenartige Doppelbindung enthaltende Verbindung auswählt unter den acyclischen oder cyclischen Olefinen mit 2 bis 20 Kohlenstoffatomen, die nicht mehr als 6 Kohlenstoffatome pro Ring enthalten.

8. Verfahren nach einem der Ansprüche 1 bis 7, dadurch gekennzeichnet, daß die eine ethylenartige Doppelbindung enthaltende Verbindung in Mengen zwischen 1,5 und 5 mol pro mol Wasserstoffperoxid eingesetzt wird.

9. Verfahren nach einem der Ansprüche 1 bis 8, dadurch gekennzeichnet, daß man es in Gegenwart eines organischen inerten Lösungsmittels ausgewählt unter primären, sekundären oder tertiären Alkoholen mit 3 bis 8 Kohlenstoffatomen durchführt.

**Claims**

1. Process for the production of epoxides, in which hydrogen peroxide is reacted with a compound containing an ethylenic double bond in a homogeneous liquid reaction mixture containing a catalyst and a base, characterised in that the concentration of water in the reaction mixture is kept below 10% by weight, a base chosen from the organic nitrogen bases with a pKa of not more than 8 is used, and a catalyst chosen from the selenium compounds is used.

2. Process according to Claim 1, characterised in that the catalyst is chosen from selenium dioxide and selenous acid.

3. Process according to either of Claims 1 and 2, characterised in that the catalyst is used in amounts of between 0.05 and 25 g per litre of the reaction mixture.

4. Process according to any one of Claims 1 to 3, characterised in that the base is chosen from the bases which are inert towards hydrogen peroxide and contain an aromatic ring in their structure.

5. Process according to Claim 4, characterised in that the base is chosen from pyridine, chloropyridine, quinoline, isoquinoline and derivatives thereof substituted by one or more alkyl groups containing 1 or 2 carbon atoms per alkyl group.

6. Process according to any one of Claims 1 to 5, characterised in that the base is used in amounts of between 0.1 and 10 moles per gram-atom of metal of the catalyst.

7. Process according to any one of Claims 1 to 6, characterised in that the compound containing an ethylenic double bond is chosen from the acyclic or cyclic olefins containing 2 to 20 carbon atoms and containing not more than 6 carbon atoms per ring.

8. Process according to any one of Claims 1 to 7, characterised in that the compound containing an ethylenic double bond is used in amounts of between 1.5 and 5 moles per mole of hydrogen peroxide.

9. Process according to any one of Claims 1 to 8, characterised in that it is carried out in the presence of an inert organic solvent chosen from the primary, secondary or tertiary alcohols containing 3 to 8 carbon atoms.

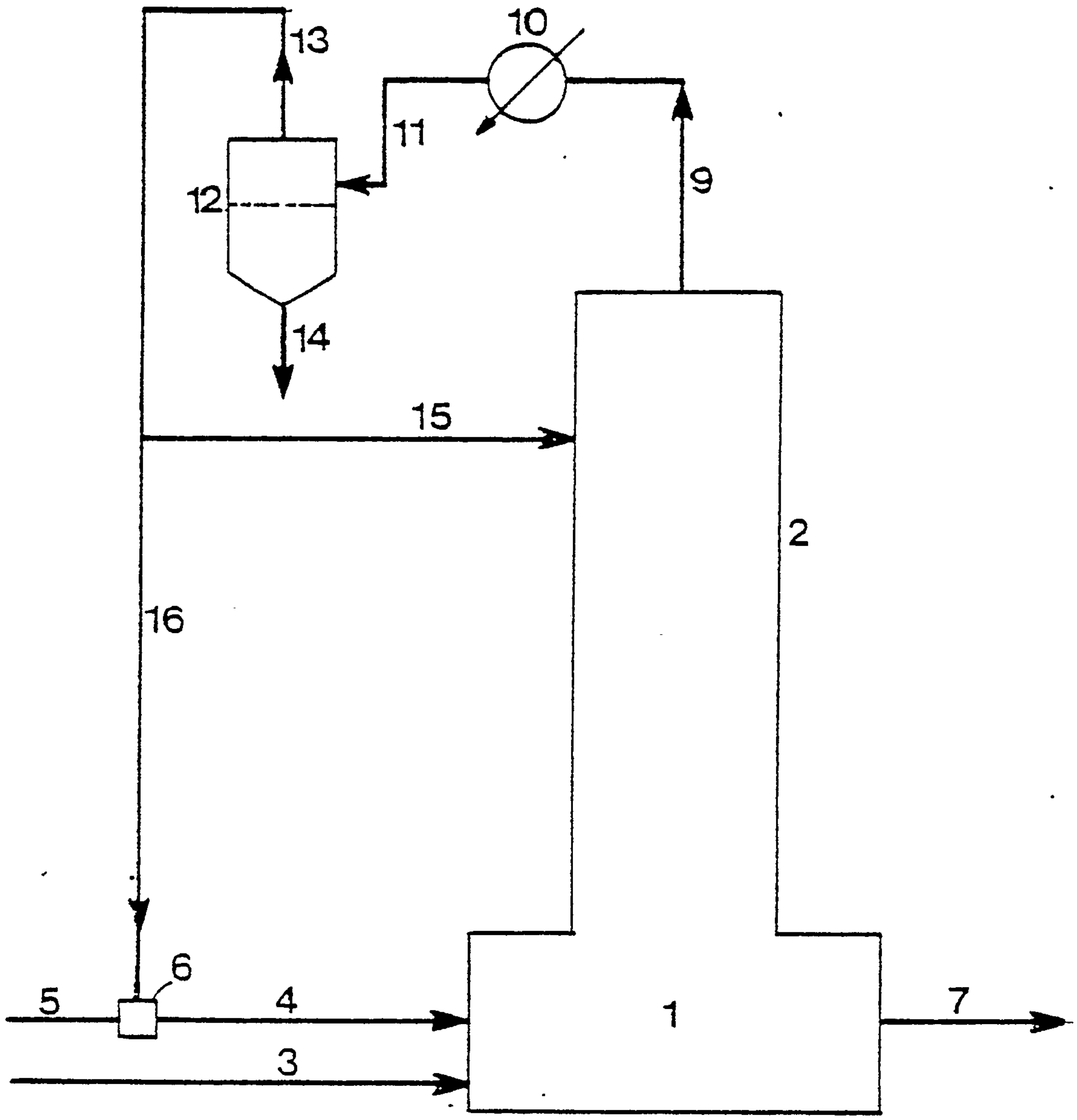
10
13
11
12
9
14
15
2
16
6
5
4
1
7
3